# EUROPÄISCHE PATENTANMELDUNG

(11) **EP 3 666 323 A1**
(43) Veröffentlichungstag der Anmeldung: **17.06.2020**
(21) Anmeldenummer: 19207411.0
(22) Anmeldetag: 06.11.2019
(51) Int. Cl.: A61M 25/02

(54) **FIXIERBAND FÜR MEDIZINISCHE INSTRUMENTE**

(30) Priorität: 12.12.2018 DE 202018107094 U
(71) Anmelder: TechniMed AG, 9400 Rorschach (CH)
(72) Erfinder: PIWONKA, Peter, 9400 Rorschach (CH)
(74) Vertreter: Körber, Martin Hans

(57) **Zusammenfassung**

Die vorliegende Erfindung betrifft ein Fixierband 10, 20 für medizinische Instrumente, wobei das Fixierband aus einem flexiblen Material mit einer Oberseite und einer Unterseite ist und eine längliche Form mit einem ersten Ende 11, 21 und einem zweiten Ende 12, 22 aufweist, und wobei im Bereiche des ersten Endes 11, 21 ein Klettmaterial 13, 23 und ein rutschhemmendes Material 14, 24 auf der Oberseite vorgesehen sind. Das rutschhemmende Material verhindert ein Verrutschen oder seitliches Herausrutschen des im Fixierband gehaltenen medizinischen Instruments.

## Beschreibung

Die vorliegende Erfindung betrifft ein Fixierband für medizinische Instrumente. Derartige Fixierbänder werden verwendet, um medizinische Instrumente, wie beispielsweise Schläuche, Drähte, Tuben und dergleichen an Patienten zu befestigen und sicher zu positionieren.

Derartige Fixierbänder sind beispielsweise aus der DE 8712347 U1 oder auch der EP 2060297 B1 bekannt. Nachteilig ist bei diesen bekannten Fixierbändern, dass sie zwar eine relativ zuverlässige Befestigung des jeweiligen medizinischen Instrumentes am Patienten ermöglichen, jedoch ein Verrutschen, beziehungsweise ein Verschieben des im Fixierband gehaltenen medizinischen Instrumentes in Bezug auf das Fixierband selber nicht zuverlässig verhindern.

Die Aufgabe der vorliegenden Erfindung besteht daher darin, ein Fixierband für medizinische Instrumente bereitzustellen, das einen sicheren und zuverlässigen Sitz des im Fixierband gehaltenen medizinischen Instrumentes gewährleistet.

Diese Aufgabe wird erfindungsgemäß durch ein Fixierband für medizinische Instrumente gemäß Anspruch 1 gelöst, wobei das Fixierband aus einem flexiblen Material mit einer Oberseite und einer Unterseite ist, und eine längliche Form mit einem ersten Ende und einem zweiten Ende aufweist, und wobei im Bereich des ersten Endes ein Klettmaterial und ein rutschhemmendes Material auf der Oberseite vorgesehen sind.

Das Vorsehen des rutschhemmenden Materials verhindert ein Verrutschen oder seitliches Herausgleiten der im Fixierband gehaltenen medizinischen Instrumente quer zur Längsrichtung des Fixierbandes. Das rutschhemmende Material kann beispielsweise durch eine Beschichtung aus Silikon oder einem anderen ähnlichen geeigneten Material gebildet sein. Das auf der Oberseite des Fixierbandes vorgesehene Klettmaterial bildet entweder mit dem Material der Oberseite selber, oder mit einem entsprechend auf der Oberseite vorgesehenen korrespondieren Klettmaterial einen Klettverschluss für das oder die medizinischen Instrumente. Das rutschhemmende Material verhindert dann ein seitliches Verrutschen der medizinischen Instrumente.

Vorteilhafterweise ist das rutschhemmende Material hierbei auf der dem ersten Ende gegenüberliegenden Seite des Klettmaterials angeordnet. Weiter vorteilhafterweise sind vom ersten Ende ausgehend zur Mitte des Fixierbandes hin auf der Oberseite jeweils abwechselnde Bereiche mit Klettmaterial und rutschhemmendem Material vorgesehen. Hierdurch können mehrere medizinische Instrumente jeweils getrennt voneinander im erfindungsgemäßen Fixierband gehalten werden. Weiter vorteilhafterweise ist das rutschhemmende Material jeweils aus mehreren einzelnen dünnen und sich quer zur Längsrichtung des Fixierbandes erstreckenden Streifen gebildet. In einer alternativen Ausgestaltung ist das rutschhemmende Material vorteilhafterweise jeweils aus zusammenhängenden Flächen gebildet.

Weiter vorteilhafterweise ist das Klettmaterial ein Hakenmaterial oder Pilzmaterial und die Oberfläche des Fixierbandes weist ein Schlaufenmaterial auf, das mit dem Hakenmaterial oder Pilzmaterial als Klettverschluss zusammenwirkt. Hierbei kann vorteilhafterweise die Oberfläche des Fixierbandes durchgehend aus dem Schlaufenmaterial bestehen. In einer alternativen Ausgestaltung weist die Oberfläche des Fixierbandes mindestens einen Bereich mit dem Schlaufenmaterial auf. Hierbei kann das Schlaufenmaterial als von der Oberfläche des Fixierbandes unabhängiges Material ausgebildet sein und entsprechend auf der Oberfläche angebracht sein.

In einer weiteren vorteilhaften Ausgestaltung ist im Bereich des ersten Endes des Fixierbandes eine Öffnung vorgesehen, durch die das zweite Ende des Fixierbandes hindurchgeführt werden kann. Auf diese Weise kann das oder die medizinischen Instrumente in der entsprechend gebildeten Schlaufe des Fixierbandes gehalten sein. Vorteilhafterweise ist hierbei das rutschhemmende Material auf der dem Klettmaterial gegenüberliegenden Seite der Öffnung vorgesehen. Weiter vorteilhafterweise ist das rutschhemmende Material zwischen dem Klettmaterial und der Öffnung vorgesehen. Weiter vorteilhafterweise ist das rutschhemmende Material zwischen der Öffnung und den jeweiligen Seitenrändern des Fixierbandes liegenden Seitenbereichen vorgesehen. Weiter vorteilhafterweise ist das Klettmaterial ein Hakenmaterial oder Pilzmaterial und auf der dem ersten Ende abgewandten Seite der Öffnung ist auf der Oberseite ein Bereich mit Schlaufenmaterial vorgesehen, das mit dem Hakenmaterial oder Pilzmaterial als Klettverschluss zusammenwirkt. Weiter vorteilhafterweise ist die Öffnung in Form eines Dreiecks gebildet, wobei eine Seite des Dreiecks rechtwinklig zur Längsachse des Fixierbandes ausgerichtet ist. Beim Einfädeln des zweiten Endes des Fixierbandes durch die Öffnung des ersten Endes kann hierdurch infolge der Dreiecksform das Fixierband durch die infolge der Dreiecksform erzeugte Kerbwirkung festgezurrt und gesichert werden, wodurch ein ungewolltes Öffnen verhindert ist. Weiter vorteilhafterweise weist der Bereich des ersten Endes des Fixierbandes mit der Öffnung eine größere Breite quer zur Längsrichtung des Fixierbandes auf als das restliche Fixierband.

Die vorliegende Erfindung wird im Folgenden anhand vorteilhafter Ausgestaltungen in Bezug auf die beigefügten Figuren näher erläutert, in denen
Figur 1 eine schematische Draufsicht einer ersten Ausgestaltung des erfindungsgemäßen Fixierbandes für medizinische Instrumente zeigt,
Figur 2 eine seitliche Ansicht einer leichten Variation des Fixierbandes von Figur 1 mit darin gehaltenen medizinischen Instrumenten zeigt, und
Figur 3 eine schematische Draufsicht auf eine weitere Ausgestaltung eines erfindungsgemäßen Fixierbandes für medizinische Instrumente zeigt.

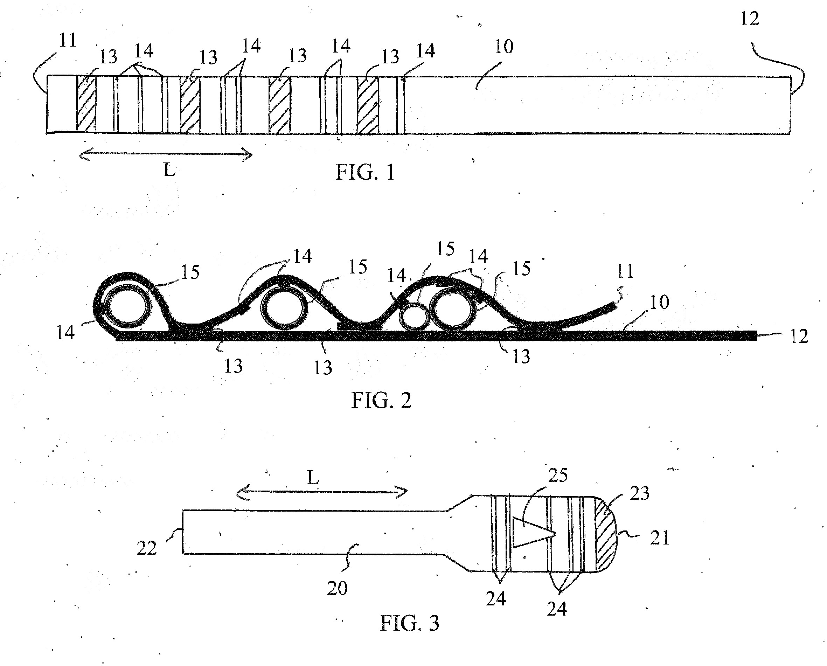

Figur 1 zeigt eine schematische Draufsicht einer ersten Ausgestaltung des erfindungsgemäßen Fixierbandes 10. In dieser Ausgestaltung hat das Fixierband 10 eine längliche Form und eine Erstreckung in Längsrichtung L, wie durch den Pfeil angedeutet, die wesentlich größer ist als die Breite des Fixierbandes (quer zur Längsrichtung L). Das Fixierband 10 weist hierbei ein erstes Ende 11 und ein zweites Ende 12 auf, sowie eine Oberseite und eine Unterseite. In der Draufsicht der Figur 1 ist die Oberseite dargestellt. In der schematischen Seitenansicht des Fixierbandes 10, die in Figur 2 dargestellt ist, sind die Oberseite und die Unterseite des Fixierbandes 10 zu erkennen. Die Oberseite des Fixierbandes 10 ist diejenige Seite, auf der Klettmaterial 13 und rutschhemmendes Material 14 angebracht, beziehungsweise angeordnet ist, und in der im zusammengeklappten Zustand, wie in Figur 2 gezeigt, die medizinischen Instrumente 15 gehalten sind. Die Unterseite des Fixierbandes 10 ist die in Figur 2 jeweils nach außen ragende Seite.

Das Fixierband 10 kann als Basismaterial beispielsweise ein Stoffband sein, oder kann auch aus jeglichem anderen geeigneten flexiblen Material wie zum Beispiel einem Kunststoffmaterial gebildet sein. Beispielsweise kann das Fixierband 10 aus einer Folie bestehen, auf die auf der Oberseite ein Flauschmaterial laminiert ist, sodass diese Oberseite im Zusammenwirken mit dem Klettmaterial 13 einen Klettverschluss bildet. In diesem Fall ist das Klettmaterial 13 durch ein entsprechendes Hakenmaterial oder Pilzmaterial (Pilzklett) gebildet. Anstelle der durchgängigen Ausbildung der Oberseite des Fixierbandes 10 als Flauschmaterial eines Klettverschlusses kann die Oberseite jedoch auch aus einem Stoffmaterial oder dergleichen bestehen, auf das jeweils zusätzliche Bereiche aus Flauschmaterial geklebt oder befestigt sind, um im Zusammenwirken mit den Haken des Klettmaterials 13 jeweils einen Klettverschluss zu bilden. Das Haken- und Flauschmaterial kann beispielsweise Nylon, Poly-Ethylen oder irgendein anderes geeignetes Material sein.

In der in den Figuren 1 und 2 dargestellten Ausgestaltung des Fixierbandes 10 sind im Bereich des ersten Endes 11, d. h. etwa im letzten Drittel oder in der letzten Hälfte des Fixierbandes zwischen der Mitte und dem ersten Ende 11, jeweils abwechselnde Bereiche mit Klettmaterial 13 und rutschhemmenden Material 14 vorgesehen. In der dargestellten Ausgestaltung erstreckt sich das Klettmaterial 13 jeweils von der einen Seite des Fixierbandes 10 und damit über die gesamte Breite. Dies ist jedoch nur ein Beispiel und das Klettmaterial 13 kann jegliche andere geeignete Form und Größe annehmen.

Weiterhin ist in der in den Figuren 1 und 2 dargestellten Ausgestaltung des Fixierbandes 10 das rutschhemmende Material 14 jeweils durch ein oder mehrere einzelne dünne und sich quer zur Längsrichtung L, über die gesamte Breite, des Fixierbandes 10 erstreckende Streifen gebildet. Alternativ kann das rutschhemmende Material 14 auch jeweils durch zusammenhängende Flächen gebildet sein. Allgemein kann das rutschhemmende Material 14 in jeglicher Form und Größe vorgesehen sein. Auch das Material des rutschhemmenden Materiales 14 kann verschiedene alternative Ausgestaltungen annehmen. Beispielsweise kann das rutschhemmende Material 14 aus einer Silikonschicht oder ähnlichem bestehen. Alternativ sind auch einzelne aufgeklebte Silikonpunkte oder irgendein anderes geeignetes Material verwendbar, das ein seitliches, d. h. quer zur Längsrichtung des Fixierbandes 10 Rutschen oder Verschieben der medizinischen Instrumente 15 verhindert.

In der Ausgestaltung des erfindungsgemäßen Fixierbandes 10, die in den Figuren 1 und 2 gezeigt ist, sind jeweils drei (Figur 2) und vier (Figur 1) abwechselnde Bereiche von Klettmaterial 13 und rutschhemmenden Material 14 gezeigt. Jegliche andere Anzahl entsprechender Bereiche ist selbstverständlich möglich, abhängig von der jeweiligen Anwendung. Weiterhin kann im Bereich des zweiten Endes 12 des Fixierbandes 10 eine zusätzliche Befestigungseinrichtung, beispielsweise in Form einer Klemme oder dergleichen, angebracht sein, mittels der das Fixierband 10 an der Kleidung eines Patienten, an einem Krankenhausbett oder dergleichen angebracht werden kann. Weiterhin kann die Unterseite des Fixierbandes 10 beispielsweise aus einem hydrophoben Material bestehen oder eine hydrophobe Beschichtung aufweisen, um das Vollsaugen des Fixierbandes 10 mit Flüssigkeiten zu verhindern.

Figur 3 zeigt eine schematische Draufsicht einer weiteren Ausgestaltung eines erfindungsgemäßen Fixierbandes 20. In Bezug auf die verwendeten Materialien und die allgemeine Form gelten alle Ausführungen, die oben in Bezug auf die Ausgestaltung des Fixierbandes 10 der Figuren 1 und 2 gemacht wurden, identisch. Auch das Fixierband 20 der Figur 3 weist eine längliche Form auf, d. h. es ist in Richtung der Längserstreckung L, siehe Pfeil in der Figur 3, wesentlich größer als in der Breite quer zur Längsrichtung L. Auch das Fixierband 20 weist ein erstes Ende 21 und ein zweites Ende 22 auf, wobei im Bereich des ersten Endes 21 ein Klettmaterial 23 angeordnet ist. Das Klettmaterial 23 wirkt mit einem entsprechenden Klettmaterial als Klettverschluss zusammen. Das entsprechende Klettmaterial ist beispielsweise die gesamte Oberseite des Fixierbandes 20, das beispielsweise als Flauschmaterial ausgeformt ist, wobei das Klettmaterial 23 dann als Hakenmaterial oder Pilzmaterial gebildet ist. Alternativ kann die Oberseite des Fixierbandes 20 an geeigneten Stellen eine Beschichtung oder dergleichen aus einem Flauschmaterial aufweisen, die mit den Haken des Klettmaterials 23 zu einem Klettverschluss zusammenwirkt, um medizinische Instrumente zu halten. Weiterhin weist das Fixierband 20 rutschhemmendes Material 24 auf, das auf der dem ersten Ende 21 des Fixierbandes 20 gegenüberliegenden Seite des Klettmateriales 23 angeordnet ist, um gehaltene medizinische Instrumente am seitlichen Rutschen zu hindern.

Der wesentliche Unterschied in der Ausgestaltung des Fixierbandes 20 im Vergleich zur Ausgestaltung des Fixierbandes 10 der Figuren 1 und 2 ist das Vorsehen einer Öffnung 25 im Bereich des ersten Endes 21. Durch diese Öffnung 25 kann das zweite Ende 22 des Fixierbandes 20 hindurchgeführt werden, um medizinische Instrumente in einer entsprechend gebildeten Schlaufe zu halten. Diese Möglichkeit bildet dann eine Alternative zum Halten der medizinischen Instrumente durch den Klettverschluss, der durch das Klettmaterial 23 und das entsprechende Schlaufenmaterial auf der Oberfläche des Fixierbandes gebildet ist. Wie in Figur 3 dargestellt, kann das rutschhemmende Material 24 an beiden Enden (in Längsrichtung L) der Öffnung 25 angeordnet sein. Zusätzlich oder alternativ ist es auch möglich, rutschhemmendes Material 24 zwischen der Öffnung und dem jeweiligen Seitenrand des Fixierbandes 20 in den Seitenbereichen vorzusehen. Die Öffnung 25 kann, wie dargestellt, in Form eines Dreiecks gebildet sein, wobei eine Seite des Dreiecks rechtwinklig zur Längsrichtung, beziehungsweise Längsachse L des Fixierbandes 20 ausgerichtet ist. Weiterhin kann der Bereich des ersten Endes 21 des Fixierbandes 20, wie in Figur 3 dargestellt, eine größere Breite quer zur Längsrichtung des Fixierbandes 20 aufweisen, als das restliche Fixierband.

Wie oben erwähnt gelten alle Ausführungen und Erläuterungen zu Materialien und Anordnung der Oberseite und Unterseite des Fixierbandes 10 der Figuren 1 und 2 in gleicher Weise für das Fixierband 20 der Figur 3. Das Gleiche gilt auch die die Materialien, Lage, Beschaffenheit und Form des Klettmaterials 13 und des rutschhemmenden Materials 14 des Fixierbandes 10 der Figuren 1 und 2, die in gleicher Weise für das Klettmaterial 23, beziehungsweise das rutschhemmende Material 24 des Fixierbandes 20 von Figur 3 gelten.

## Patentansprüche

1. Fixierband (10, 20) für medizinische Instrumente,
wobei das Fixierband aus einem flexiblen Material mit einer Oberseite und einer Unterseite ist und eine längliche Form mit einem ersten Ende (11, 21) und einem zweiten Ende (12, 22) aufweist, und
wobei im Bereiche des ersten Endes (11, 21) ein Klettmaterial (13, 23) und ein rutschhemmendes Material (14, 24) auf der Oberseite vorgesehen sind.

2. Fixierband (10, 20) für medizinische Instrumente gemäß Anspruch 1,
wobei das rutschhemmende Material auf der dem ersten Ende (11, 21) gegenüberliegenden Seite des Klettmaterials (13, 23) angeordnet ist.

3. Fixierband (10, 20) für medizinische Instrumente gemäß Anspruch 1 oder 2,
wobei vom ersten Ende (11, 21) ausgehend zur Mitte des Fixierbandes (10, 20) hin auf der Oberseite jeweils abwechselnde Bereiche mit Klettmaterial (13, 23) und rutschhemmendem Material (14, 24) vorgesehen sind.

4. Fixierband (10, 20) für medizinische Instrumente gemäß einem der Ansprüche 1 bis 3, wobei das rutschhemmende Material (14, 24) jeweils aus mehreren einzelnen dünnen und sich quer zur Längsrichtung des Fixierbandes (10, 20) erstreckenden Streifen gebildet ist.

5. Fixierband (10, 20) für medizinische Instrumente gemäß einem der Ansprüche 1 bis 3, wobei das rutschhemmende Material (14, 24) jeweils aus zusammenhängenden Flächen gebildet ist.

6. Fixierband (10, 20) für medizinische Instrumente gemäß einem der Ansprüche 1 bis 5, wobei das Klettmaterial (13, 23) ein Hakenmaterial oder Pilzmaterial ist und die Oberfläche des Fixierbandes (10, 20) ein Schlaufenmaterial aufweist, das mit dem Hakenmaterial oder Pilzmaterial als Klettverschluss zusammenwirkt.

7. Fixierband (10, 20) für medizinische Instrumente gemäß Anspruch 6,
wobei die Oberfläche des Fixierbandes (10, 20) durchgehend aus dem Schlaufenmaterial besteht.

8. Fixierband (10, 20) für medizinische Instrumente gemäß Anspruch 6,
wobei die Oberfläche des Fixierbandes (10, 20) mindestens einen Bereich mit dem Schlaufenmaterial aufweist.

9. Fixierband (20) für medizinische Instrumente gemäß einem der Ansprüche 1 bis 8,
wobei im Bereich des ersten Endes (21) eine Öffnung (25) vorgesehen ist, durch die das zweite Ende (22) hindurch geführt werden kann.

10. Fixierband (20) für medizinische Instrumente gemäß Anspruch 9,
wobei das rutschhemmende Material (24) auf der dem Klettmaterial (13, 23) gegenüber liegenden Seite der Öffnung (25) vorgesehen ist.

11. Fixierband (20) für medizinische Instrumente gemäß Anspruch 9 oder 10,
wobei das rutschhemmende Material (24) zwischen dem Klettmaterial und der Öffnung (25) vorgesehen ist.

12. Fixierband (20) für medizinische Instrumente gemäß einem der Ansprüche 9 bis 11,
wobei das rutschhemmende Material (24) an zwischen der Öffnung (25) und den jeweiligen Seitenrändern des Fixierbandes (20) liegenden Seitenbereichen vorgesehen ist.

13. Fixierband (20) für medizinische Instrumente gemäß einem der Ansprüche 9 bis 12,
wobei das Klettmaterial ein Hakenmaterial oder Pilzmaterial ist und auf der dem ersten Ende (21) abgewandten Seite der Öffnung (25) auf der Oberseite ein Bereich mit Schlaufenmaterial vorgesehen ist, das mit dem Hakenmaterial oder Pilzmaterial als Klettverschluss zusammenwirkt.

14. Fixierband (20) für medizinische Instrumente einem der Ansprüche 9 bis 13,
wobei die Öffnung (25) in Form eines Dreiecks gebildet ist, wobei eine Seite des Dreiecks rechtwinkelig zur Längsachse des Fixierbandes (20) ausgerichtet ist.

15. Fixierband (20) für medizinische Instrumente einem der Ansprüche 9 bis 14,
wobei der Bereich des ersten Endes (21) des Fixierbandes mit der Öffnung (25) eine größere Breite quer zur Längsrichtung des Fixierbandes (20) als das restliche Fixierband aufweist.
